# FASCICULE DE BREVET EUROPEEN

(11) **EP 2 529 685 B1**
(45) Date de publication et mention de la délivrance du brevet: **16.04.2014**
(21) Numéro de dépôt: 12169176.0
(22) Date de dépôt: 24.05.2012
(51) Int. Cl.: A61B 17/80

(54) **Ensemble comprenant une pièce implantable destinée à être fixée à un ou plusieurs os ou à des parties d'os à réunir, et au moins une vis de fixation de cette pièce implantable à cet ou ces os**
Einheit, die ein implantierbares Teil zur Befestigung an einem oder mehreren Knochen oder an zu vereinenden Knochenteilen umfasst, und mindestens eine Schraube zur Fixierung dieses implantierbaren Teils an diesem/diesen Knochen
Assembly comprising an implantable part to be secured to one or more bones or bone parts to be joined, and at least one screw for securing said implantable part to said one or more bones

(30) Priorité: 31.05.2011 FR 1154791
(43) Date de publication de la demande: 05.12.2012
(73) Titulaire: Compagnie Financière et Médicale, 01000 Bourg en Bresse (FR)
(72) Inventeur: Martin, Jean-Jacques, 01000 BOURG EN BRESSE (FR)
(74) Mandataire: Jeannet, Olivier

(56) Documents cités:
- US-A1- 2008 140 130
- US-A1- 2008 208 259
- US-A1- 2010 312 286

## Description

La présente invention concerne un ensemble comprenant une pièce implantable destinée à être fixée à un ou plusieurs os ou à des parties d'os à réunir, et au moins une vis de fixation de cette pièce implantable à cet ou ces os. Cette pièce implantable peut notamment être une embase de fixation à un os d'une partie d'une prothèse d'articulation, en particulier une embase de fixation d'une glène à une omoplate dans une prothèse totale de l'épaule ; cette pièce implantable peut également être une plaque corticale ou toute autre pièce d'un matériel d'arthrodèse ou d'ostéosynthèse.

Il est bien connu d'utiliser des vis pour fixer des pièces implantables telles que des embases servant à fixer des composants de prothèses d'articulation ou des plaques corticales ou similaires servant à réaliser une arthrodèse ou une ostéosynthèse d'un ou plusieurs os.

Une embase ou une plaque très classique comprend des trous cylindriques lisses dans lesquels les vis de fixation sont destinées à être engagées. Chaque trou peut être ajusté à la dimension de la vis destinée à être engagée dans ce trou, définissant ainsi une direction unique d'implantation de cette vis, ou peut être d'un diamètre supérieur à celui du corps de la vis, pour permettre l'implantation de cette vis selon une pluralité de directions d'implantation.

Dans le premier cas, l'embase ou la plaque présente l'inconvénient d'imposer une direction d'implantation à la vis et donc d'interdire des directions d'implantation autres que celle qui est imposée. Or, selon l'anatomie de l'os ou l'état du patient, il peut être préféré d'implanter la vis selon une direction différente de celle du trou. Dans le deuxième cas, l'embase ou la plaque n'exclut pas un positionnement défectueux de la vis par rapport à l'os ou aux os dans lesquels elle est implantée, en particulier le risque d'un débouché de la vis au niveau d'une surface articulaire d'un ou plusieurs de ces os.

Il est également connu d'aménager un taraudage dans le trou et un filetage sur la tête de la vis, le filetage de la vis venant en engagement avec le taraudage du trou. De tels ensembles ont également pour inconvénient d'imposer à la vis une direction d'implantation déterminée par ledit engagement.

Par ailleurs, il existe sur de tels ensembles un risque notable de dévissage d'une ou plusieurs vis au cours du temps sous l'effet des efforts répétés subis par le ou les os. Pour remédier à ce risque de dévissage, il a été conçu divers systèmes de rétention axiale des vis, qui, d'une manière générale, présentent les inconvénients d'être relativement onéreux à fabriquer et de ne pas être toujours très efficaces.

Les publications de demandes de brevet N° US 2010/312286, US 2008/208259 et US 2008/140130 illustrent diverses techniques selon l'art antérieur.

La présente invention vise à remédier aux inconvénients précités.

Son objectif principal est de fournir un ensemble tel que précité, dans lequel au moins une vis puisse être implantée selon une direction d'implantation choisie parmi plusieurs directions d'implantation possibles, et dans lequel le risque de dévissage de cette vis au cours du temps soit réduit.

Un autre objectif de l'invention est de fournir un ensemble dans lequel le risque d'un positionnement défectueux d'une vis soit exclu, tout en conservant une possibilité de choix parmi plusieurs directions d'implantation possibles.

Dans l'ensemble concerné, de manière connue en soi, ladite pièce implantable comprend au moins un trou taraudé et la tête de la vis destinée à être engagée dans ce trou est filetée.

Selon l'invention,
- le trou est de forme cylindrique et comprend au moins une encoche radiale débouchant au moins dans la face proximale de ladite pièce implantable, c'est-à-dire dans la face de cette pièce implantable par laquelle la vis est destinée à être engagée dans le trou, cette encoche radiale réalisant une interruption du taraudage que comprend le trou ;
- la tête de la vis est de forme conique, ayant un angle au sommet compris entre 15 et 25°, et
- le pas du filet de la tête de la vis est compris entre le tiers et la moitié du pas du filet formant le taraudage du trou, et la profondeur du filet de cette tête est comprise entre la moitié et les deux tiers de la profondeur du pas du filet formant le taraudage du trou.

L'inventeur a ainsi pris le contre-pied des enseignements élémentaires de la mécanique, en cherchant à engager et coincer une tête de vis de forme différente de celle du trou destiné à recevoir cette vis. Il a pu déterminer que la combinaison des caractéristiques précitées permettait d'obtenir le double résultat avantageux de (i) rendre possible l'engagement de la vis selon une pluralité de directions d'implantation et (ii) de réaliser, en fin de vissage, un coincement efficace de la tête de la vis dans le trou, de nature à s'opposer à tout risque de dévissage. La pluralité de directions d'implantation possibles de la vis résulte (i) de la présence de ladite encoche, qui interrompt localement le taraudage du trou et qui permet une légère déformation de ladite pièce implantable au niveau de ce trou, (ii) de la forme conique spécifique de la tête, et (iii) de l'absence de venue étroitement en prise des filets respectifs de la tête de la vis et du trou au cours d'une première partie de la phase de vissage ; ledit coincement efficace résulte quant à lui également (i) de ladite possibilité de légère déformation permise par l'encoche, (ii) de la forme conique spécifique de la tête, qui permet la venue progressive des filets respectifs de la tête de la vis et du trou en imbrication au cours de la deuxième partie de la phase de vissage, et qui réalise cette imbrication complète en fin de vissage.

De préférence, ledit angle au sommet est de 20°.

Le pas du filet de la tête de la vis est quant à lui de préférence de l'ordre de la moitié du pas du filet du taraudage du trou.

Ces valeurs ont pu être déterminées comme étant optimales pour l'obtention de ladite pluralité de directions d'implantation et du coincement de la vis. Elles permettent une possibilité d'angulation de l'axe de la vis par rapport à l'axe du trou pouvant atteindre 20°.

Cet axe du trou peut lui-même ne pas être parallèle à un plan de référence de la pièce implantable, par exemple le plan général dans lequel s'étend une plaque corticale. Cette angulation du trou vient ainsi s'ajouter à l'angulation de la vis rendue possible par ce trou pour permettre une angulation importante de la vis par rapport audit plan général.

De préférence, le filet à os que comprend le corps de la vis s'étend le long de ce corps jusqu'à proximité immédiate de la tête de cette vis, en dessous de cette tête, de telle sorte que l'extrémité proximale de ce filet soit située, lorsque la vis est en place sur ladite pièce implantable, à l'intérieur dudit trou.

La présence de ce filet dans le trou contribue à réaliser un coincement de la vis dans le trou

De préférence, ladite encoche s'étend sur l'ensemble de la hauteur du trou.

Ladite légère déformation de la pièce implantable au niveau de cette encoche est ainsi pleinement rendue possible.

De préférence, le trou comprend une pluralité d'encoches.

Selon une possibilité, ces encoches peuvent être présentes sur l'ensemble de la circonférence du trou, permettant une orientation angulée de la vis sur l'ensemble de cette circonférence. Elles peuvent notamment être régulièrement réparties sur cette circonférence.

Selon une autre possibilité, ces encoches sont présentes sur un premier secteur de la circonférence du trou et absentes sur un deuxième secteur de la circonférence de ce trou.

Une orientation angulée de la vis est ainsi interdite sur le côté du trou opposé à celui sur lequel se trouve ledit deuxième secteur. Par cette possibilité d'angulation sélective d'une vis, résultant de l'aménagement des encoches en des emplacements judicieux des trous, il peut être réalisé une pièce implantable permettant de s'assurer contre le risque d'une implantation défectueuse d'une vis, en particulier le risque d'un débouché d'une vis au niveau d'une surface articulaire d'un ou plusieurs des os traités.

Cette possibilité peut notamment être mise en oeuvre sur une embase de glène de prothèse d'épaule, pour orienter une vis d'ancrage dans le pilier de la coracoïde et dans le pilier de l'omoplate. Cette possibilité peut également être mise en oeuvre sur des plaques corticales dont une extrémité est destinée à être implantée à proximité d'une surface articulaire que forme un os, en particulier sur une plaque humérale supérieure ou une plaque radiale inférieure.

L'invention sera bien comprise, et d'autres caractéristiques et avantages de celle-ci apparaîtront, en référence au dessin schématique annexé, représentant, à titre d'exemples non limitatifs, plusieurs formes de réalisation possibles de l'ensemble pièce implantable-vis qu'elle concerne.
La figure 1 est une vue en perspective d'une plaque comprenant une série de trous dans lesquels sont destinées à être reçues des vis d'ancrage de cette plaque ;
la figure 2 est une vue en perspective de l'un des trous, à échelle agrandie;
la figure 3 est une vue du trou, de face ;
la figure 4 est une vue d'une vis de côté ;
la figure 5 est une vue en perspective de la tête de cette vis, à échelle agrandie ;
la figure 6 est une vue de cette tête de côté, à échelle agrandie;
les figures 7 à 9 sont des vues partielles de la plaque et de la vis en coupe, au cours de trois phases successives de mise en place de la vis dans le trou ;
la figure 10 est une vue développée du trou, avec indication schématique de la position d'une spire du filet de la vis selon trois angulations, montrant une absence de saut de filet ;
la figure 11 est une vue antérieure, avec coupe, d'une partie d'une omoplate dans laquelle a été implantée une embase de réception d'une glène ;
la figure 12 est une vue sagittale de la partie d'omoplate, selon l'axe de ladite embase ;
les figures 13 à 18 sont des vues de face de six formes de réalisation possibles d'un trou que comprend la pièce implantable selon l'invention ;
les figures 19 et 20 sont des vues respectivement sagittale et antérieure de la partie supérieure d'un humérus sur laquelle a été mis en place une plaque conforme à l'invention ;
la figure 21 est une vue de dessus d'une plaque pour l'extrémité inférieure d'un radius, et
les figures 22 et 23 sont des vues respectivement de dessus et de côté de cette plaque, après mise en place sur un radius.

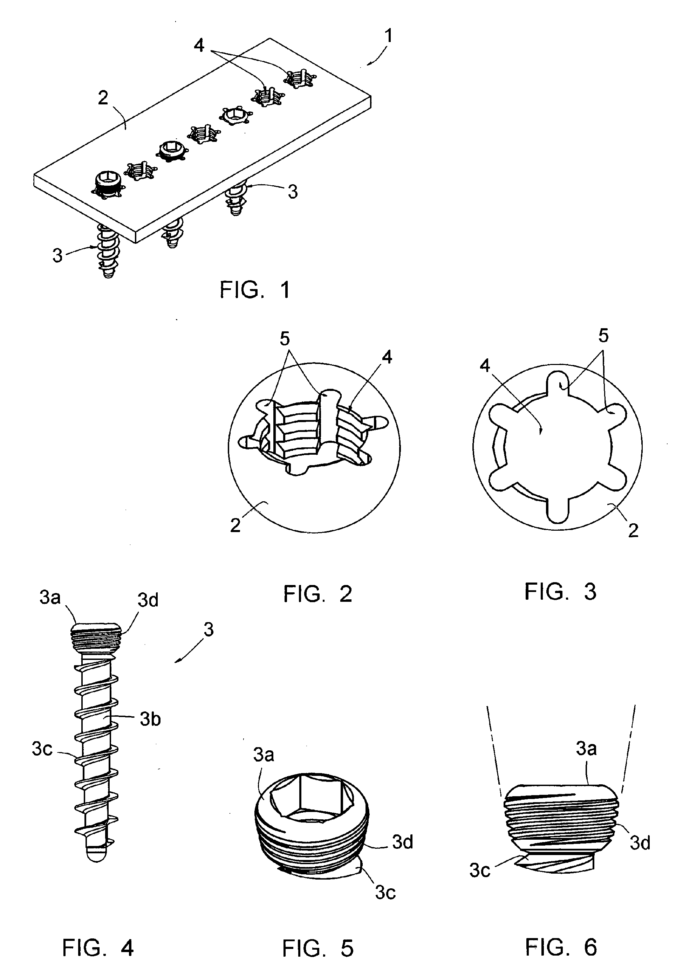

La figure 1 représente un ensemble 1 comprenant une pièce implantable 2 destinée à être fixée à un ou plusieurs os ou à des parties d'os à réunir, et plusieurs vis 3 de fixation de cette pièce implantable 2 à cet ou ces os.

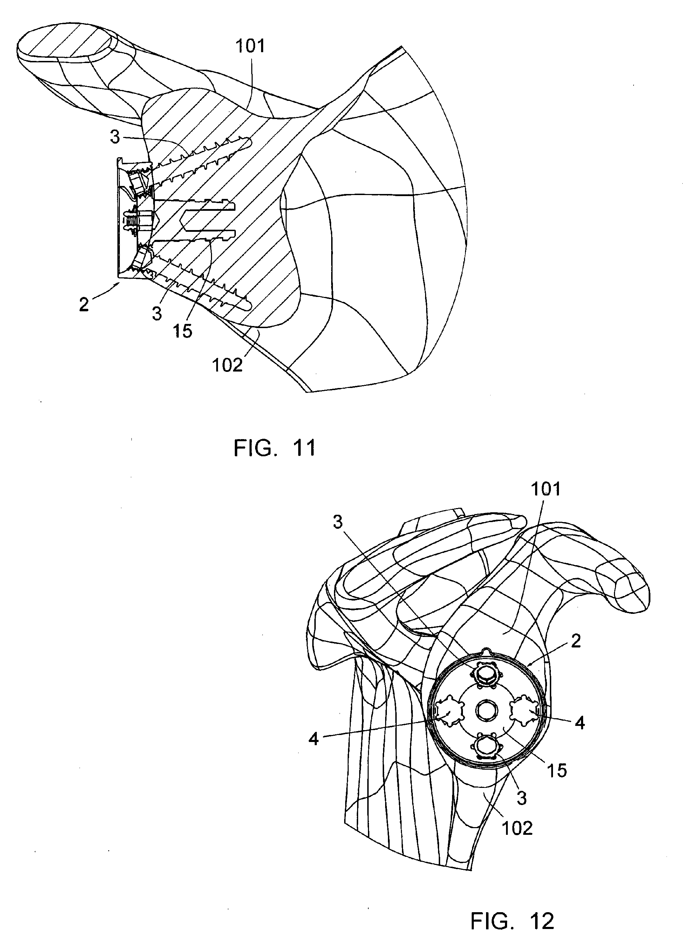

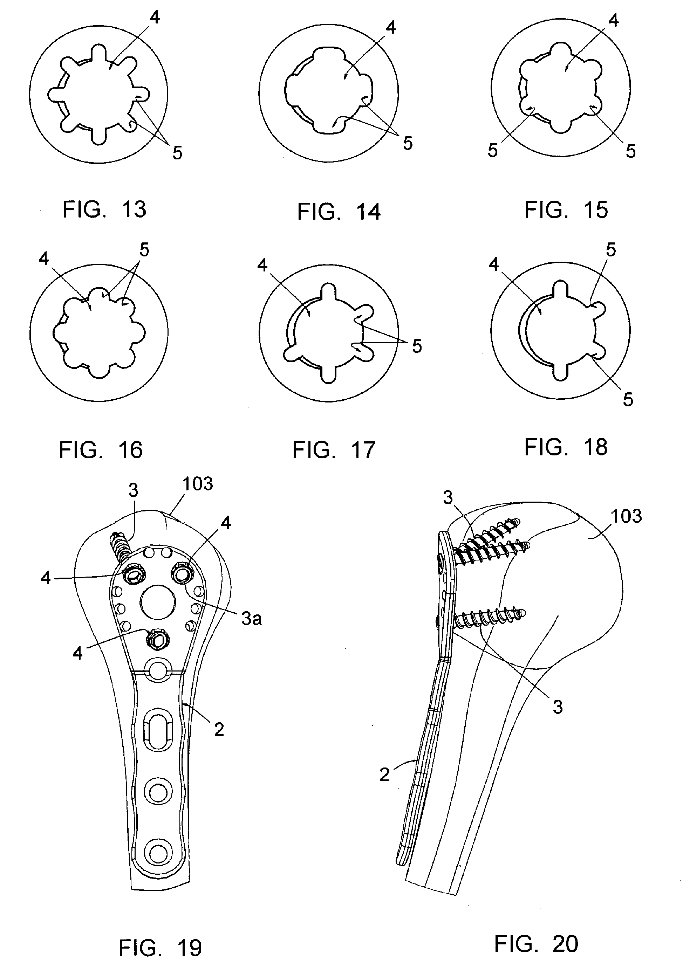

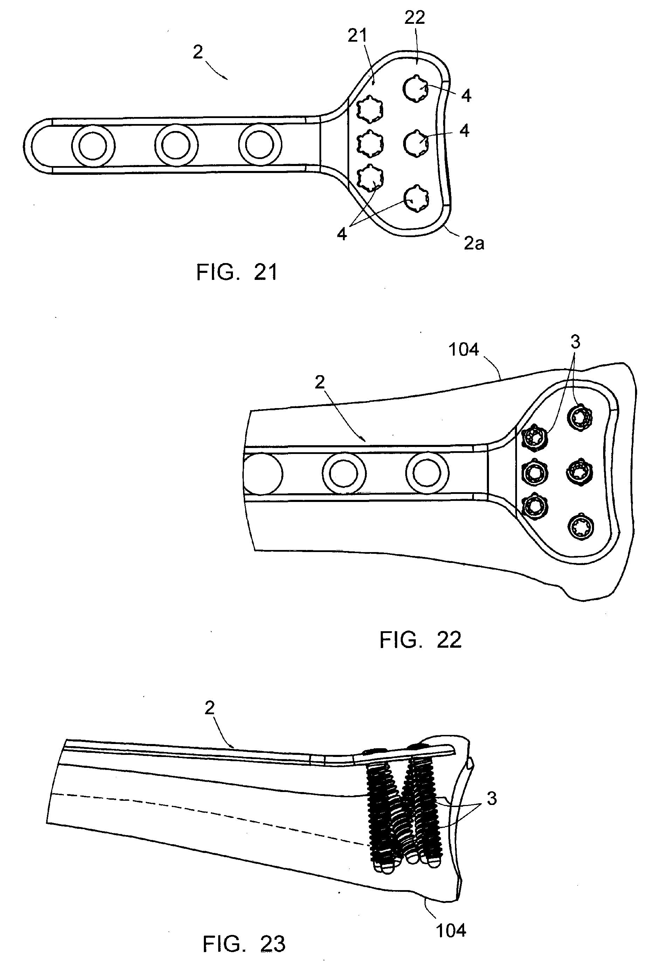

Dans le but d'une illustration purement schématique, la pièce implantable 2 a été représentée sur la figure 1 comme étant une plaque rectangulaire de forme tout à fait quelconque. Il apparaît sur les figures 11 et 12 que cette pièce implantable peut être une embase 2 de fixation à une omoplate 100 d'une glène ou d'une pièce formant une surface articulaire convexe dite "métaglène" ou "glénosphère" (non représentée) ; cette pièce implantable peut également être une plaque corticale humérale 2 (cf. figures 19 et 20) ou plaque corticale pour l'extrémité inférieure du radius (cf. figures 21 à 23).

La plaque 2 représentée sur la figure 1 comprend une pluralité de trous 4 alignés, dont chacun est destiné à recevoir une vis 3.

Comme le montrent plus particulièrement les figures 2 et 3, chaque trou 4 est cylindrique et taraudé, et comprend six encoches 5 s'étendant radialement, régulièrement réparties sur sa circonférence. Ces encoches 5 s'étendent sur l'ensemble de l'épaisseur de la plaque 2 et interrompent par conséquent le filet du taraudage du trou 4 sur l'ensemble de la hauteur de ce dernier. Elles ont une forme substantiellement en "U", c'est-à-dire que chacune d'elles forme deux faces radiales en regard l'une de l'autre et un fond arrondi raccordant ces deux faces.

En référence aux figures 4 à 6, il apparaît que chaque vis 3 comprend une tête 3a conique et filetée, et un corps 3b pourvu d'un filet 3c adapté à une prise d'appui dans un os.

L'angle au sommet du cône définissant la tête 3a est de 20° (cf. figure 6). Le pas du filet de cette tête 3a est de l'ordre du tiers du pas du taraudage d'un trou 4, et la profondeur de ce filet est de l'ordre de la moitié de celle du pas du taraudage d'un trou 4.

Le filet 3d de la tête 3a est symétrique, présente des flancs pouvant être inclinés entre 45° et 75° et a une profondeur identique sur toute la longueur de la tête. Le filet peut être à double entrée.

Le filet à os 3c que comprend le corps 3b de la vis s'étend le long de ce corps jusqu'à proximité immédiate de la tête 3a, et s'interrompt immédiatement en dessous de cette tête 3a.

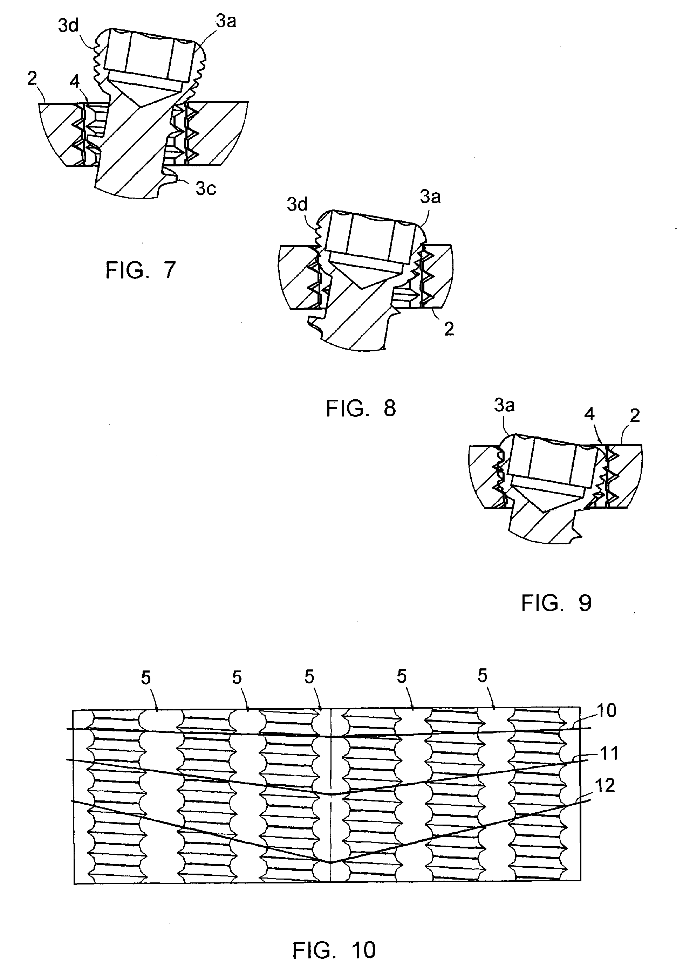

Chaque vis 3 est destinée à être engagée dans un trou 4 et à être vissée dans le ou les os auxquels la plaque 2 et destinée à être fixée. Les vis 3 peuvent être engagées dans les trous 4 perpendiculairement à la plaque 2 comme le montre la figure 1 ou être mises en place dans ces trous 4 de manière non perpendiculaire à la plaque 2, ainsi que cela apparaît sur les figures 7 à 9.

Cette possibilité d'angulation d'une vis 3, et donc d'une pluralité de directions d'implantation possibles de cette vis, résulte (i) de la présence des encoches 5, qui interrompent localement le taraudage du trou 4 et qui permettent une légère déformation de la pièce 2 au niveau de ce trou 4, (ii) de la forme conique précitée de la tête 3a, et (iii) de l'absence de venue étroitement en prise des filets respectifs de la tête 3a de la vis 3 et du trou 4 au cours d'une première partie de la phase de vissage, à savoir sensiblement jusqu'au niveau de la position de la vis 3 montrée sur la figure 8.

À partir de cette position, et sur une deuxième partie de la phase de vissage allant jusqu'à la position de fin de vissage montrée sur la figure 9, il se réalise un coincement progressif et efficace de la tête 3a dans le trou 4, de nature à s'opposer à tout risque de dévissage de la vis 3. Ce coincement résulte (i) de ladite possibilité de légère déformation permise par les encoches 5, et (ii) de la forme conique précitée de la tête 3a, qui permet la venue progressive des filets respectifs de la tête 3a et du trou 4 en imbrication au cours de ladite deuxième partie de la phase de vissage, et qui réalise cette imbrication complète en fin de vissage.

Il se comprend également en référence à la figure 9 qu'une portion de la première spire du filet à os 3c est à même de se trouver, dans cette position de fin de vissage, à l'intérieur du trou 4, contribuant ainsi au coincement de la vis 3 dans ce trou 4.

La figure 10 représente le développé d'un trou 4 avec trois lignes 10, 11, 12 représentant chacune le développé d'une spire du filet 3d de la tête 3a, pour des angulations respectives de la vis 3 de 0°, 6° et 12°. Elle montre qu'il n'y a pas de sauf de filet.

En référence aux figures 11 et 12, il apparaît que l'embase 2 précitée comprend un plot central d'ancrage 15 et quatre trous 4 répartis autour de ce plot 15, destinés à recevoir des vis 3 (seulement deux des quatre vis 4 sont mises en place sur l'embase 2 représentée, en cours de pose).

Il apparaît que les axes des trous 4 ne sont pas parallèles à l'axe du plot 15 mais inclinés par rapport à cet axe, de 10° dans l'exemple représenté. Cette angulation des trous 4 vient ainsi s'ajouter à l'angulation des vis 3 rendue possible par ces trous, de manière à permettre une angulation importante des vis 3 par rapport à un plan de référence perpendiculaire à l'axe du plot 15. Dans l'exemple représenté, l'angulation totale de la vis 3 inférieure est de 20° (10° d'angulation du trou 4 par rapport audit axe, et 10° d'angulation de la vis 3 dans le trou 4). Grâce à cette possibilité d'angulation importante, la vis 3 supérieure et la vis 3 inférieure peuvent être orientées de façon optimale pour un ancrage respectivement dans le pilier 101 de la coracoïde et dans le pilier 102 de l'omoplate.

Les figures 13 à 16 montrent des trous 4 présentant des formes et des nombres d'encoches 5 différents de ceux décrits ci-dessus. Le trou 4 représenté sur la figure 13 comprend des encoches identiques à celles décrites plus haut, mais en comprend huit au lieu de six. Le trou 4 représenté sur la figure 14 comprend seulement quatre encoches 5, également en forme de "U" mais beaucoup plus larges que celles décrites plus haut. Le trou 4 représenté sur la figure 15 comprend six encoches 5 à section en demi-cercle. Le trou 4 représenté sur la figure 16 comprend huit de telles encoches 5 à section en demi-cercle.

Le trou 4 représenté sur la figure 17 comprend cinq encoches 5 régulièrement aménagées sur un premier secteur de l'ordre de 240° et est dépourvu d'encoche sur un deuxième secteur de l'ordre de 120°. De manière similaire, le trou 4 représenté sur la figure 18 comprend quatre encoches 5 régulièrement aménagées sur un premier secteur de l'ordre de 180° et est dépourvu d'encoches sur un deuxième secteur de l'ordre de 180°. L'absence d'encoches 5 sur lesdits deuxièmes secteurs se traduit par une rigidité de la pièce implantable 2 et par une continuité du filet du taraudage du trou 4 au niveau de ces deuxièmes secteurs, dont il résulte une impossibilité d'orienter une vis 3 sur les côtés des trous 4 opposés à ceux sur lesquels se trouvent ces deuxièmes secteurs.

Il peut ainsi être conçu des pièces implantables 2 avec lesquelles le risque d'une implantation défectueuse d'une vis 3 est éliminé.

Ainsi, en référence à la figure 19, il apparaît qu'une plaque humérale 2 peut comprendre, dans sa portion épiphysaire, deux trous 4 côte à côte dont les secteurs tournés du côté opposé à l'extrémité épiphysaire de cette plaque 2 sont dépourvus d'encoches. Dès lors, une orientation excessive des vis 3 vers la corticale osseuse épiphysaire de la tête humérale 103 est empêchée, comme visible sur la figure 20, ce qui prévient le risque d'un débouché de ces vis 3 au niveau de la face articulaire de l'humérus. Le troisième trou 4 que comprend ladite portion épiphysaire présente également un secteur dépourvu d'encoches sur le côté de ce trou opposé à extrémité épiphysaire de la plaque 2 ; ce secteur prévient ainsi l'orientation de la vis 3 engagée dans ce trou vers à diaphyse humérale. Par contre, la possibilité reste entière de choisir la direction d'implantation des vis 3 dans les autres directions que mentionnées ci-dessus.

La figure 21 montre une plaque 2 pour une extrémité inférieure du radius, présentant classiquement une portion distale 2a élargie. Dans cette portion distale 2a sont aménagés transversalement une série proximale 21 de quatre trous 4 et une série distale 22 de trois trous 4. Les trous 4 de la série proximale 21 comprennent chacun six encoches telles que décrites plus haut, permettant une orientation multidirectionnelle des vis 3. Chaque trou 4 de la série distale 22 comprend un premier secteur présentant quatre encoches 5, tourné vers l'extrémité distale de la plaque 2, et un deuxième secteur dépourvu d'encoches, situé sur le côté du trou 4 tourné du côté de ladite série proximale 21.

Comme cela se comprend en référence aux figures 22 et 23, les vis 3 engagées dans les trous 4 de la série distale 22 ne peuvent pas être orientées en direction de l'extrémité distale du radius 104, ce qui prévient tout risque de débouché de ces vis 3 au niveau des surfaces articulaires que comprend ce radius 104.

Il apparaît de ce qui précède que l'invention fournit un ensemble 1 incluant une pièce implantable 2 et au moins une vis 3, ayant pour avantages déterminants de rendre possible l'engagement de la vis 3 dans un trou 4 de la pièce 2 selon une pluralité de directions d'implantation, et de réaliser, en fin de vissage, un coincement efficace de la tête 3a de la vis dans le trou 4, de nature à s'opposer à tout risque de dévissage. Un autre avantage déterminant de cet ensemble 1 est de permettre d'éliminer le risque d'un positionnement défectueux d'une vis 3.

L'ensemble 1 selon l'invention est utilisable sur toutes les anatomies autres que celles précitées, telles que fémur, tibia, coude ou pied, et fonctionne quel que soit le matériau utilisé, par exemple inox ou titane.

L'invention a été décrite ci-dessus en référence à des formes de réalisation données à titre d'exemples. Il va de soi qu'elle n'est pas limitée à ces formes de réalisation mais qu'elle s'étend à toutes les autres formes de réalisations couvertes par les revendications ci-annexées. En particulier, l'embase 2 montrée sur les figures 11 et 12 pourrait comprendre des trous 4 médians (c'est-à-dire les deux trous 4 autres que le trou supérieur et le trou inférieur visibles sur la figure 12) dépourvus d'encoches 5 sur leurs secteurs tournés vers l'extérieur de l'embase 2, interdisant ainsi une orientation des vis dans des directions d'implantation risquant de faire déboucher ces vis en dehors de l'os.

## Revendications

1. Ensemble (1) comprenant une pièce implantable (2) destinée à être fixée sur un ou plusieurs os (101 à 104) ou à des parties d'os à réunir, et au moins une vis (3) de fixation de cette pièce implantable (2) à ce ou ces os, ladite pièce implantable (2) comprenant au moins un trou (4) taraudé et la tête (3a) de la vis (3) destinée à être engagée dans ce trou (4) étant filetée ;
**caractérisé en ce que** :
- le trou (4) est de forme cylindrique et comprend au moins une encoche radiale (5) débouchant au moins dans la face proximale de ladite pièce implantable (2), c'est-à-dire dans la face de cette pièce implantable (2) par laquelle la vis (3) est destinée à être engagée dans le trou (3), cette encoche radiale (5) réalisant une interruption du taraudage que comprend le trou (4) ;
- la tête (3a) de la vis (3) est de forme conique, ayant un angle au sommet compris entre 15 et 25°, et
- le pas du filet de la tête (3a) de la vis (3) est compris entre le tiers et la moitié du pas du filet formant le taraudage du trou (4), et la profondeur du filet de cette tête est comprise entre la moitié et les deux tiers de la profondeur du pas du filet formant le taraudage du trou (4).

2. Ensemble (1) selon la revendication 1, **caractérisé en ce que** ledit angle au sommet est de 20°.

3. Ensemble (1) selon la revendication 1 ou la revendication 2, **caractérisé en ce que** le pas du filet de la tête (3a) de la vis (3) est de l'ordre de la moitié du pas du filet du taraudage du trou (4).

4. Ensemble (1) selon l'une des revendications 1 à 3, **caractérisé en ce que** le filet à os (3c) que comprend le corps (3b) de la vis (3) s'étend le long de ce corps (3b) jusqu'à proximité immédiate de la tête (3a) de cette vis, en dessous de cette tête, de telle sorte que l'extrémité proximale de ce filet (3c) soit située, lorsque la vis (3) est en place sur ladite pièce implantable (2), à l'intérieur dudit trou (4).

5. Ensemble (1) selon l'une des revendications 1 à 4, **caractérisé en ce que** ladite encoche (5) s'étend sur l'ensemble de la hauteur du trou (4).

6. Ensemble (1) selon l'une des revendications 1 à 5, **caractérisé en ce que** le trou (4) comprend une pluralité d'encoches (5).

7. Ensemble (1) selon la revendication 6, **caractérisé en ce que** lesdites encoches (5) sont présentes sur l'ensemble de la circonférence du trou (4), permettant une orientation angulée de la vis (3) sur l'ensemble de cette circonférence.

8. Ensemble (1) selon la revendication 6, **caractérisé en ce que** lesdites encoches (5) sont présentes sur un premier secteur de la circonférence du trou (4) et absentes sur un deuxième secteur de la circonférence de ce trou.

9. Ensemble (1) selon l'une des revendications 1 à 8, **caractérisé en ce que** ladite pièce implantable (2) est une embase de fixation à un os d'une partie d'une prothèse d'articulation, en particulier d'une embase (2) de fixation d'une glène, ou d'une pièce formant une surface articulaire convexe, à une omoplate dans une prothèse totale de l'épaule.

10. Ensemble (1) selon l'une des revendications 1 à 8, **caractérisé en ce que** ladite pièce implantable (2) est une plaque corticale, en particulier une plaque humérale supérieure ou une plaque radiale intérieure.

## Patentansprüche

1. Einheit (1), die ein implantierbares Teil (2) zur Befestigung an einem oder mehreren Knochen (101 bis 104) oder an zu vereinenden Knochenteilen umfasst, und mindestens eine Schraube (3) zur Fixierung dieses implantierbaren Teils (2) an diesem/diesen Knochen, wobei das implantierbare Teil (2) mindestens eine Bohrung (4) mit Innengewinde umfasst und der Kopf (3a) der Schraube (3), die dazu bestimmt ist, in die Bohrung (4) eingedreht zu werden, ein Gewinde aufweist;
**dadurch gekennzeichnet, dass**: :
- die Bohrung (4) eine zylindrische Form aufweist und mindestens eine radiale Einkerbung (5) umfasst, die mindestens in die proximale Fläche des implantierbaren Teils (2) einmündet, also in die Fläche des implantierbaren Teils (2), durch die die Schraube (3) in die Bohrung (4) einzuführen ist, wobei die radiale Einkerbung (5) eine Unterbrechung des Innengewindes der Bohrung (4) erzeugt;
- der Kopf (3a) der Schraube (3) eine konische Form mit einem Scheitelwinkel im Bereich zwischen 15 und 25° aufweist, und
- die Gewindesteigung des Kopfs (3a) der Schraube (3) im Bereich zwischen einem Drittel und der Hälfte der Gewindesteigung des Innengewindes der Bohrung (4) liegt, und die Gewindetiefe des Kopfs im Bereich zwischen der Hälfte und zwei Dritteln der Gewindetiefe des Innengewindes der Bohrung (4) liegt.

2. Einheit (1) nach Anspruch 1, **dadurch gekennzeichnet, dass** der Scheitelwinkel 20° beträgt.

3. Einheit (1) nach Anspruch 1 oder Anspruch 2, **dadurch gekennzeichnet, dass** die Gewindesteigung des Kopfs (3a) der Schraube (3) in der Größenordnung der Hälfte der Gewindesteigung des Innengewindes der Bohrung (4) liegt.

4. Einheit (1) nach einem der Ansprüche 1 bis 3, **dadurch gekennzeichnet, dass** das Knochengewinde (3c), das der Schaft (3b) der Schraube (3) umfasst, sich entlang des Schafts (3b) bis zu einer unmittelbaren Nähe zum Kopf (3a) der Schraube unterhalb des Kopfes erstreckt, so dass sich das proximale Ende des Gewindes (3c) im Inneren der Bohrung (4) befindet, wenn die Schraube (3) auf dem implantierbaren Teil (2) platziert ist.

5. Einheit (1) nach einem der Ansprüche 1 bis 4, **dadurch gekennzeichnet, dass** die Einkerbung (5) sich über die gesamte Tiefe der Bohrung (4) erstreckt.

6. Einheit (1) nach einem der Ansprüche 1 bis 5, **dadurch gekennzeichnet, dass** die Bohrung (4) eine Vielzahl Einkerbungen (5) umfasst.

7. Einheit (1) nach Anspruch 6, **dadurch gekennzeichnet, dass** die Einkerbungen (5) auf dem gesamten Umfang der Bohrung (4) vorhanden sind, wobei sie eine abgewinkelte Orientierung der Schraube (3) auf dem gesamten Umfang ermöglichen.

8. Einheit (1) nach Anspruch 6, **dadurch gekennzeichnet, dass** die Einkerbungen (5) in einem ersten Abschnitt des Umfangs der Bohrung (4) vorhanden sind und in einem zweiten Abschnitt des Umfangs der Bohrung nicht vorhanden sind.

9. Einheit (1) nach einem der Ansprüche 1 bis 8, **dadurch gekennzeichnet, dass** das implantierbare Teil (2) eine Basis zur Fixierung eines Teils einer Gelenkprothese an einem Knochen ist, insbesondere eine Basis (2) zur Fixierung einer Gelenkpfanne oder eines Teils, das eine konvexe Gelenkoberfläche bildet, an einem Schulterblatt in einer Schultertotalprothese.

10. Einheit (1) nach einem der Ansprüche 1 bis 8, **dadurch gekennzeichnet, dass** das implantierbare Teil (2) eine kortikale Platte ist, insbesondere eine obere Humerusplatte oder eine innere Radiusplatte.

## Claims

1. Assembly (1) comprising an implantable part (2) designed to be fastened to one or more bones (101 to 104) or bone portions to be joined, and at least one screw (3) for fastening the implantable part (2) to this or these bones, said implantable part (2) comprising at least one tapped hole (4), and the head (3a) of the screw (3) designed to be engaged in said hole (4) is threaded ;
**characterized in that**:
- the hole (4) is cylindrical and comprises at least one radial notch (5) emerging at least in the proximal surface of said implantable part (2), i.e in the surface of said implantable part (2) through which the screw (3) is designed to be engaged in the hole (3), said radial notch (5) producing an interruption in the tapping comprised by the hole (4);
- the head (3a) of the screw (3) is conical, having an apical angle comprised between 15 and 25°, and
- the pitch of the thread of the head (3a) of the screw (3) is comprised between one third and one half the pitch of the thread making up the tapping of the hole (4), and the depth of the thread of that head is comprised between one half and two thirds of the depth of the pitch of the thread making up the tapping of the hole (4).

2. Assembly (1) according to claim 1, **characterized in that** said apical angle is 20°.

3. Assembly (1) according to claim 1 or claim 2, **characterized in that** the pitch of the thread of the head (3a) of the screw (3) is in the vicinity of one half the pitch of the thread of the tapping of the hole (4).

4. Assembly (1) according to any of claims 1 to 3, **characterized in that** the bone thread (3c) that the body (3b) of the screw (3) comprises extends along that body (3b) into the immediate vincinity of said head (3a) of said screw, below said head, such that the proximal end of said screw thread (3c) is situated, when the screw (3) is in place on said implantable part (2), inside said hole (4).

5. Assembly (1) according to any of claims 1 to 4, **characterized in that** said notch (5) extends over the entire height of said hole (4).

6. Assembly (1) according to any of claims 1 to 5, **characterized in that** the hole (4) comprises a plurality of said notches (5).

7. Assembly (1) according to claim 6, **characterized in that** said notches (5) are present over the entire circumference of said hole (4), allowing an angulated orientation of said screw (3) over the entire circumference.

8. Assembly (1) according to claim 6, **characterized in that** said notches (5) are present on a first sector of the circumference of the hole (4) and absent on a second sector on the circumference of said hole.

9. Assembly (1) according to one of claims 1 to 8, **characterized in that** said implantable part (2) is a fastening base for fastening a part of a joint prosthesis to a bone, especially a fastening base (2) for a glenoid part, or for a part forming a convex articular surface, to a scapula in a total shoulder prosthesis.

10. Assembly (1) according to one of claims 1 to 8, **characterized in that** said implantable part (2) is a cortical plate, in particular a superior humeral plate or an inferior radius plate.
